# EUROPEAN PATENT APPLICATION

(11) **EP 4 752 897 A1**
(43) Date of publication of application: **03.06.2026**
(21) Application number: 24216132.1
(22) Date of filing: 28.11.2024
(51) Int. Cl.: G16H 20/30, G16H 50/20, A61B 5/11

(54) **HUMAN POSE EVALUATION**

(71) Applicant: Hitachi, Ltd., Tokyo 100-8280 (JP)
(72) Inventor: AKIYAMA, Takayuki, Tokyo, 100-8280 (JP)
(74) Representative: Mewburn Ellis LLP

(57) **Abstract**

A system for performing postural evaluation. The system comprises: a posture determination unit configured to receive motion and/or positional data relating to one or more parts of a user's body to determine a posture of the user; a task determination unit configured to use the determined posture and/or motion and/or positional data to determine a task being performed by the user; a posture evaluation unit configured to: retrieve a predetermined template posture corresponding to the determined task, and measure a difference between the determined posture of the user and the predetermined template posture; and a posture optimizer unit configured to: perform a mitigation action when the measured difference is greater than the a predetermined posture difference threshold.

## Description

### Field of the Invention

The present invention relates to a system, computer-implemented method and non-transitory computer-readable storage medium for human pose evaluation.

### Background

In contemporary work environments, employees frequently engage in tasks that predispose them to a spectrum of posture-related health complications. These range from minor annoyances like muscle aches to more debilitating conditions such as chronic back pain and musculoskeletal disorders, significantly affecting both productivity and quality of life. Traditional methods for addressing these risks have encompassed ergonomic evaluations, physical therapy, and educational initiatives aimed at promoting correct posture. Yet, these approaches largely depend on an individual's self-awareness and their capacity for self-correction, a consistency that many find difficult to sustain over time.

Existing systems are capable of monitoring a user's spinal alignment in real-time, and issuing alerts when poor posture is detected. However, these systems fall short as they signal only when slouching or similar poor postures are observed, without offering adequate guidance for users to rectify their posture comprehensively. Further, such systems do not take into account the specific conditions and reasons for why the user may have adopted a particular posture, and so fail to take a holistic view of the user and provide more tailored advice.

The present invention has been devised in light of the above considerations.

### Summary of the Invention

In a first aspect, the present invention provides a system for performing postural evaluation, the system comprising: a posture determination unit configured to receive motion and/or positional data relating to one or more parts of a user's body to determine a posture of the user; a task determination unit configured to use the determined posture and/or motion and/or positional data to determine a task being performed by the user; a posture evaluation unit configured to: retrieve a predetermined template posture corresponding to the determined task, and measure a difference between the determined posture of the user and the predetermined template posture; and a posture optimizer unit configured to: perform a mitigation action when the measured difference is greater than the a predetermined posture difference threshold.

By taking into consideration the particular task being performed by the user when performing the posture evaluation, the system is better able to determine whether or not the user's posture is within an acceptable tolerance compared to an ideal posture (e.g., the predetermined template posture), and therefore whether or not corrective action is necessary. As such, the system is able to better protect the user from posture-related health risks.

The posture evaluation unit may be configured, instead of retrieving a predetermined template posture corresponding to the determined task and measuring the difference between the predetermined posture of the user and the predetermined template posture, to measure a difference between the determined posture of the user and a predetermined template posture. In such examples, the posture optimizer unit may be configured to retrieve a predetermined posture difference threshold corresponding to the determined task, and perform the mitigation action when the measured difference is greater than this predetermined posture difference threshold (which corresponds to the determined task).

The system may be for performing postural evaluation in a workplace setting (e.g., an office setting). The postural evaluation may be performed at or around a workstation in that the system may be configured to receive motion and/or positional data relating to one or more parts of a user who is in a workplace setting. The workstation may be any type of desk used by a worker. For example, an office desk, or an engineering bench. Alternatively, the workstation may refer to a volume of space in which a user typically works, such as a space within a car factory within which a user works on engines.

The system may be configured to obtain the motion and/or positional data relating to the one or more parts of the user's body from a sensor arrangement.

Parts of the user's body may refer to any portion of the body, including, though not limited to, the head, shoulders, upper body (e.g., torso), lower body (e.g., legs), upper arms, forearms, and hands.

The various 'units' referred to within the first aspect of the present invention (e.g., 'posture determination unit', 'posture evaluation unit', 'task determination unit') may refer to blocks of software configured to perform the functions associated with that specific unit. Each block of software may be functionally independent from the other blocks, and/or may be performed at a different geographic location (for example, at a different point on a CPU chip, or by a different computing device altogether). The blocks of software may be performed at different times and in different orders that would be recognisable to the skilled person from the present disclosure.

Alternatively, the software associated with each unit may comprise a single larger piece of software, in which one or more units perform their functions or portions of their functions at the same time.

The units may all be contained on a computing device, such as a personal computer or a mobile phone. The computing device may be communicatively connectable to a sensor arrangement so as to transfer data from the sensor arrangement to the computing device. For example, via a wired or wireless connection. Alternatively, a sensor arrangement may form part of the computing device.

'Motion data' includes at least one of speed data and acceleration data. The motion data may include both a size and direction of the motion. Motion data may relate to the detection of general motion, and/or specific motions. A specific motion being a specific movement of a portion of the user's body. The specific motion may be a single action, or a single repetitive action repeated at least two times. An example may be the rhythmic twisting of a user's wrist when screwing in a screw.

'Positional data' of portions of the user's body may include absolute positional data, or relative positional data. Relative positional data may be relative to a fixed predetermined point (e.g., an anchor point, it being determined, for example, by a sensor arrangement on first activation), and/or relative to one or more other portions of the user's body measured by a sensor arrangement. In the latter example, the relative positional data relating to each portion of the user's body may be determined relative to a single portion of the body, or specific portions of the user's body may be determined relative to other specific portions of the user's body. For example, the position of the forearm may be determined relative to the upper arm.

The 'posture' of the user may refer to an overall posture adopted by the user. In particular, whether the user is standing, sitting or crouching, and the specific pose they adopt within one of these positions (e.g., when sitting, are they hunched over a keyboard). Alternatively or additionally, the posture may relate to one or more specific components of a pose adopted by a user. These specific components may relate to only one or a few parts of the user's body (e.g., their upper body), and/or the arrangement of particular parts of the user's body relative to one another.

The posture may be determined based on joint angles between different parts of the user's body. For example, the angle of the upper arm relative to the forearm (i.e., 'elbow bend'), or the angle of the lower body relative to the torso. Alternatively or additionally, the posture may be determined based on other features, such as the angle/curvature of the back, how the user's neck/head is tiled, and/or whether the user has their elbow resting on a desk. These features are calculated by the posture determination unit using the positional and/or motion data.

The predetermined template posture may be configured to represent an ideal or preferred posture of the user. The predetermined template posture may be a combination of ideal or preferred positions of one or more parts of the user's body, wherein the position of each of the one or more parts of the user's body may be defined relative to the position of one or more other parts of the user's body. In some examples, the predetermined template posture is represented as a combination of ideal or preferred (e.g., joint) angles between different parts of the user's body.

There may be a different predetermined template posture for each task. Alternatively, two or more tasks may have the same predetermined template posture.

The predetermined template postures may be stored in a predetermined template posture database. The predetermined template posture database may also store the tasks associated with each predetermined template posture database.

The 'task' can be an activity, job or action. The task can be a particular administrative task that the user is performing on a computer whilst sat at their desk. The task may be a data entry task. The task may involve entering customer information (e.g., repeatedly inputting customer details into a database or a customer relationship management, CRM, system), or inventory logging (e.g., entering product information, quantities, and stock levels into inventory management software).

Alternatively, the task can be a more physical task, such as where the user is screwing in a screw using a screwdriver. The task may involve the user performing a repetitive action (e.g., a repetitive twisting motion of the wrist when screwing in a screw). The task may have been assigned to the user (e.g., by the present system). The user may be assigned multiple tasks to be completed in a particular order.

The stored posture and/or stored motion and/or stored positional data corresponding to stored tasks may be stored in a task determination database.

Each task may have a different corresponding predetermined template posture.

The difference between the determined posture and the predetermined template posture measured by the posture evaluation unit can refer to/be based on one or more specific components of a posture. As discussed above, components of a posture may refer to specific portions of the body, and/or the arrangement of specific portions of a body relative to one another. Differences between individual components of the posture may be used to determine an overall difference between the determined posture and the predetermined template posture. Different components of the posture may be weighted differently when determining the overall difference between the determined posture and the predetermined template posture. For example, elbow bend may be more important than head tilt, and therefore a large difference between the elbow bend of the determined posture and the elbow bend of the predetermined template posture may lead to a very large determined overall difference between the determined posture and predetermined template posture even when the head tilt of the determined posture and the head tilt of the predetermined template posture are identical or close to identical.

The predetermined posture difference threshold may define a posture range around the predetermined template posture that is considered an acceptable level of deviation from the predetermined template posture. If the difference between the determined posture and the predetermined template posture is less than the predetermined posture difference threshold, then no mitigation action is taken. If the difference between the determined posture and the predetermined template posture is greater than the predetermined posture difference threshold, then mitigation action is taken.

The predetermined posture difference threshold may include an overall difference threshold relating to the overall difference between the determined posture and predetermined template posture (see above for discussion on overall difference). Alternatively or additionally, the predetermined posture difference threshold may include one or more specific component difference thresholds associated with specific components of the posture (e.g., to do with only one or a few parts of the body, and/or the arrangement of particular parts of the body relative to one another). In other words, the predetermined posture difference threshold may comprise a plurality of thresholds relating to different comparisons of the determined posture and the predetermined template posture (e.g., the overall difference and one or more specific component differences).

The predetermined posture difference threshold may vary depending on the determined task. E.g., different tasks may have corresponding predetermined posture difference thresholds with different threshold magnitudes (e.g., different sized ranges of acceptable posture). The differences between different predetermined posture difference thresholds may include differences in an overall difference threshold of each respective predetermined posture difference threshold, and/or may include differences in one or more specific component thresholds of each respective predetermined posture difference threshold (overall difference thresholds and specific component difference thresholds are discussed above).

'Mitigation action' is taken to mean that the system takes direct and/or indirect steps to improve the posture of the user.

The mitigation action taken can depend on the size of the difference between the predetermined template posture and the determined posture. More specifically, the mitigation can depend on how far outside the predetermined posture difference threshold that the determined posture of the user falls. For example, when the determined posture of the user is only just outside the predetermined posture difference threshold, the mitigation action may be gentle. Contrastingly, when the determined posture of the user is far from the predetermined posture difference threshold, the mitigation action may be more severe. The severity of the mitigation action may increase in discreet steps at discreet intervals of distance from the predetermined posture difference threshold. The severity of the mitigation action taken, or at least how quickly the severity of the mitigation action taken is increased with distance from the predetermined posture difference threshold may vary by task. Advantageously, this allows the mitigation action taken to be kept proportionate to the risk posed to the user in remaining in an incorrect posture whilst performing a particular task.

In some examples, the mitigation action includes notifying the user that the measured difference is greater than the predetermined posture difference threshold. Advantageously, this allows the user to take immediate and direct action to correct their posture.

The notification may comprise a visual message. For example, the notification may flash a colour or warning sign at the user, or may show the user customized text. The customized text may take the form of different reminder types. For example, the reminder types may include gentle, encouraging and positive, instructional, motivational, health benefit highlight, friendly nudge, humorous or interactive reminder types. The specific reminder types used may depend on the severity of the mitigation required and/or the determined task. In examples where the severity of the action is increased for any reason (see above), the mitigation action may go from a gentle reminder to a motivational reminder

Alternatively or additionally, the notification may include haptic and/or sound elements. Additional elements may be added to the notification as the required severity of the mitigation action increases.

The notification may be generated using a generative model. The generative model may generate the notification based on predefined notification settings. The generative model may be a large language model (LLM). The large language model may be pretrained using a large set of training data. The training data may include appropriate text responses to incorrect or deficient postures, and/or text labelled to indicate the tone of the text.

In some examples, the mitigation action further includes providing guidance to the user as to how to improve their posture. More specifically, the system may provide guidance to the user as to how they can bring their posture into line with the predetermined template posture, or at least within the predetermined posture difference threshold of the predetermined template posture.

The system may provide recommendations and/or instructions for how the user should modify their posture. These may be in the form of customized text, or a pictorial representation of what the predetermined template posture looks like.

Advantageously, this facilitates quicker posture correction for the user.

In some examples, the mitigation action includes reassigning or causing the reassignment of a series of tasks to be performed by the user.

The reassignment may include selecting a new task with the aim of improving/changing the posture of the user (that is, the new task may be selected on the basis of the measured difference).

For example, the reassignment may comprise swapping a first task that the user is currently performing for a second, different, task. The first task the user is currently performing may be a difficult posture task, and the second task may be an easy (or easier, than the current task) posture task. Alternatively or additionally, the reassignment may comprise reassigning a series of difficult posture tasks arranged one after another so that one or more easy posture tasks are arranged in between. For example, an easy posture task may be arranged between each pair of adjacent difficult posture tasks.

Difficult posture tasks may be tasks where the predetermined template posture is difficult to maintain for extended periods (for example because it causes large degrees of strain on the body over time), and/or where small deviations from the predetermined template posture cause a large risk of injury or other long term problems for the user (for example, where small deviations from the predetermined template posture cause large strains on the body). Easy posture tasks may be defined as the opposite of difficult posture tasks.

The system may be configured to obtain motion and/or positional data relating to one or more parts of respective users' bodies (e.g., from a plurality of sensor arrangements, which may or may not form part of the system). The posture determination unit may be configured to determine, for each of the users, the respective posture using the respectively collected data. The posture evaluation unit may be configured to measure a respective difference for each user between the respectively determined posture and a respective predetermined template posture. The task determination unit may be configured to use the respectively determined posture and/or motion and/or positional data to determine a respective task being performed by each user. The posture optimizer unit may be configured to retrieve a respective predetermined posture difference threshold corresponding to each determined task, and perform a respective mitigation action for the relevant user when their corresponding measured difference is greater than their respective predetermined posture difference threshold. The system may be configured to perform this in parallel (i.e. simultaneously for each monitored user), or sequentially (and so checking one user before moving to the next, possibly in a repeating loop).

In examples where there is more than one user, reassignment may involve swapping tasks between users. For example, where one user has a series of easy posture tasks, and a second user has a series of difficult posture tasks, the system may reassign some of the difficult posture tasks of the second user to the first user, and some of the easy posture tasks of the first user to the second user.

The reassignment may be implemented by transmitting a request to a work allocation module, the work allocation module being configured to assign tasks to one or more users. Upon receipt of the request, the work allocation module may reassign a series of tasks assigned to the user. This can be implemented, for example, by transmitting a reassignment message to a device utilised by the relevant users (e.g., smartphone, terminal etc).

In some examples, the system further comprises a or the sensor arrangement(s) to obtain the motion and/or positional data.

The sensor arrangement may comprise one or more sensors. The sensors may be optical sensors and/or accelerometer-based sensors (e.g., inertial measurement unit, IMU, sensors).

The sensor arrangement may comprise two or more subset sensor arrangements, wherein each subset sensor arrangement may comprise one or more sensors. Each of the one or more sensors/subset sensor arrangements may be configured to obtain a specific type of data. For example, position data, motion data, or another form of data which is usable to derive position data and/or motion data. Alternatively, each of the one or more sensors/subset sensor arrangements may be usable to obtain both position and motion data. In this example, the one or more sensors/subset sensor arrangements may measure a first type of data (e.g., motion or position) directly, and may then determine a second type of data (e.g., motion or position) using the first type of data. For example, motion data may be determined from position data by monitoring changes to the position data over time, or position data may be determined from motion data by using the motion data to determine changes to a position from a starting point over time. Where one type of data is determined from another type of data (e.g., motion data from time series position data) this determination can be performed by a controller which forms either part of the system or the sensor arrangement.

Each of the one or more sensors/subset sensor arrangements may be configured to obtain (e.g., position and/or motion) data on or of a specific part of the user's body. For example, a first sensor/sensor arrangement may be configured to obtain data on the user's head, a second sensor/sensor arrangement may be configured to obtain data on a user's torso, a third sensor/sensor arrangement may be configured to obtain data on a user's upper left or right arm, a fourth sensor/sensor arrangement may be configured to obtain data on a user's lower left or right arm (i.e., forearm), etc.

In some examples, the sensor arrangement comprises one or more sensors attached to the user. Each of the one or more sensors attached to the user may collect positional and/or motion data on the respective portion/body part of the user they are attached to.

The one or more sensors may be attached to an item of clothing of the user.

The one or more sensors may be accelerometer-based sensors (e.g., Inertial Measurement Unit, IMU, sensors).

In examples where there is more than one sensor attached to the user, said attached sensors may be attached to different body parts of the user.

In some examples, the sensor arrangement comprises one or more optical sensors.

The one or more optical sensors may be unattached (separate) from the user. The one or more optical sensors may be configured to point at the user.

In examples where there is more than one optical sensor, each of the more than one optical sensors may be arranged to point toward different regions of space. Said different regions of space may correspond to the likely positions of different parts of the user's body when arranged in front of the sensor arrangement. In this way, different sensors may be configured to obtain motion/positional data on different parts of the user's body.

Further in examples where there is more than one optical sensor, each of the more than one optical sensors may be arranged around an expected occupation space of the user (e.g., at equidistant points around the expected occupation space). Advantageously, this allows data to be collected on different portions of the user's body. However, the present invention is not limited in this way, and the sensors may instead be grouped in a single cluster.

In some examples, the one or more optical sensors include at least one camera. For example, an environmental camera.

In some examples, the task detection unit determines the task using a pretrained action recognition model, the pretrained action recognition model having been trained using historical motion and/or positional data linked to corresponding tasks.

The pretrained action recognition model may be a trained machine learning model. More particularly, the action recognition model may use a trained neural network.

The action recognition model may be continuously (re-)trained whilst in use to improve the task determination units recognition of prestored tasks and/or to incorporate new tasks into its list of recognised tasks. The continuous (re-)training may use feedback from a user. Feedback may include an indication of what task they have been performing, whether the posture they adopted whilst performing a task was correct and/or whether they felt any strain or pain during the performance of the task, whether their movements during the performance of the task are characteristic of the performance of that task, which part of the body is most associated with the performance of a task etc. Alternatively or additionally, the action recognition model may be self-learning.

In some examples, to determine the task being performed by the user, the task determination unit and action recognition model may compare one or more of the determined posture and/or obtained motion and/or obtained positional data to stored postures and/or stored motion and/or stored positional data which have been predetermined to correspond to particular recognised tasks. If the determined posture and/or obtained motion and/or obtained positional data matches, or substantially matches (e.g., within a predetermined matching threshold), the stored posture and/or stored motion and/or stored positional data corresponding to a particular task, the task determination unit determines said particular task is the task being performed by the user.

Where the determined posture and/or obtained motion and/or obtained positional data is determined to substantially match (e.g., within a predetermined matching threshold) multiple sets of stored posture and/or stored motion and/or stored positional data corresponding to multiple stored tasks, the task determination unit may determine that the task being performed is the stored task of the multiple stored task whose corresponding stored posture and/or stored motion and/or stored positional data matches the determined posture and/or motion and/or positional data most closely.

Where the determined posture and/or obtained motion and/or obtained positional data is determined not to substantially match (e.g., within a predetermined matching threshold) the stored posture and/or stored motion and/or stored positional data corresponding to any of the stored tasks, the task determination unit may return a 'fallback' task as the task being performed by the user, said fallback task having a fallback predetermined template posture. Said fallback predetermined template posture may be configured to represent a standard posture that may be adopted by a user to prevent posture-related injury.

The task determination unit may also utilise a work schedule to confirm the task being performed. This can include identifying the user, and looking up an assigned task for the identified user.

In some examples, the posture determination unit and/or the task determination unit are respectively configured to continuously re-determine the posture and task. Advantageously, this allows real-time feedback to be provided to the user on the state of their posture, and whether it requires correction.

In some examples, the system is further configured to vary the mitigation action if the measured difference between the determined posture of the user and the predetermined template posture does not decrease over time. More specifically, the severity/urgency of the mitigation action may be increased if the measured difference between the determined posture of the user and the predetermined template posture does not decrease over time.

The difference between the determined posture of the user and the predetermined template posture not decreasing over time includes examples where the difference between the determined posture of the user and the predetermined template posture remains the same over time, as well as examples where the difference between the determined posture of the user and the predetermined template posture increases over time. In the latter example, the severity/urgency of the mitigation may be increased more quickly and/or to a greater degree.

The system may be further configured to vary (e.g., increase the severity/urgency of) the mitigation action where the difference between the determined posture of the user and the predetermined template posture does not decrease at a great enough rate over time.

The system may be configured to: assess whether the posture has improved over time (e.g., has the difference between the determined posture and the predetermined template posture decreased), determine the size of the improvement of the posture, compare the size of the improvement of the posture to a predetermined improvement amount, and perform a mitigation action if the improvement of the posture is less than the predetermined improvement amount.

In some examples, the posture determination unit and/or task determination unit are respectively configured to determine the posture and task using data collected over a predetermined interval of time.

Said predetermined interval of time may correspond to motion and/or positional data collected over a plurality of time steps. The motion and/or positional data collected over the plurality of time steps may be averaged to determine an approximate of the user's posture over the plurality of time steps. Advantageously, this allows partial damping/filtering out of sudden jumps or starts in the user posture which are quickly rectified, thus preventing overuse of the mitigation actions.

In some examples, the predetermined template posture is one of a plurality of predetermined template postures corresponding to different tasks, and the posture evaluation unit is configured to retrieve the predetermined template posture corresponding to the determined task

This advantageously provides an increased degree of flexibility in the system, since it allows the system to be used to monitor and evaluate a user's posture even if the task requires the user to adopt an unusual posture.

In some examples, the system further comprises a chatbot unit configured to provide coaching advice to the user based on the user's motivation.

The user's motivation may be determined by measuring the difference between a determined posture of the user and a predetermined template posture at a first time and a second time later than the first time, and subsequently comparing the measured differences at the first time and the second time to determine whether the user's posture is improving or not.

The coaching advice may include generated content (e.g., text), wherein the tone of the generated content depends on the user's motivation. The tone of the generated text may be, for example: gentle, motivational, comedic, instructional, serious or encouraging.

In some examples, the posture evaluation unit and the task determination unit perform their determinations simultaneously or substantially simultaneously. This advantageously increases the speed at which the system is able to react to and mitigate poor user posture, therefore reducing the risk posed to the user.

However, the present disclosure is not limited in this way. For example, the posture may be calculated first, or the task may be calculated first.

Further, when the task is not being determined based on the determined posture, the posture detection unit and the task detection unit can perform their determinations substantially simultaneously.

According to a second aspect of the present invention, there is provided a computer-implemented method for performing postural evaluation, the method comprising the steps of: receiving motion and/or positional data relating to one or more parts of a user's body from a sensor arrangement; determining a posture of the user using the motion and/or positional data; determining a task being performed by the user using the determined posture and/or motion and/or positional data; retrieving a predetermined template posture corresponding to the determined task; measuring a difference between the determined posture of the user and the predetermined template posture; and performing a mitigation action when the measured difference is greater than the a predetermined posture difference.

As above, the method of the second aspect may include a step, instead of measuring the difference between the determined posture of the user and the predetermined template posture, of measuring a difference between the predetermined posture of the user and a predetermined template posture. In such examples, the retrieving step would include, instead of retrieving a predetermined template posture, retrieving a predetermined posture difference threshold corresponding to the determined task, and then performing the mitigation action when the measured difference is greater than the predetermined posture difference threshold.

In some examples, the computer-implemented method may further comprise the step of using the sensor arrangement to obtain the motion and/or positional data.

According to a third aspect of the present invention, there is provided a non-transitory computer-readable storage medium containing machine-executable instructions which, when executed on a processor, cause the processor to perform the method of the second aspect.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Fig. 1** is a schematic of a system for posture evaluation according to a first example of the invention.
**Fig. 2** is a flowchart for performing posture evaluation.
**Fig. 3** is a table illustrating an example of a predetermined posture difference threshold.
**Fig. 4** is a schematic of a system according to a second example of the invention.
**Fig. 5** is a schematic of a system according to a third example of the invention.
**Fig. 6** is a schematic of a system according to a fourth example of the invention.

### Detailed Description of the Invention

Aspects and embodiments of the present invention will now be discussed with reference to the accompanying figures. Further aspects and embodiments will be apparent to those skilled in the art.

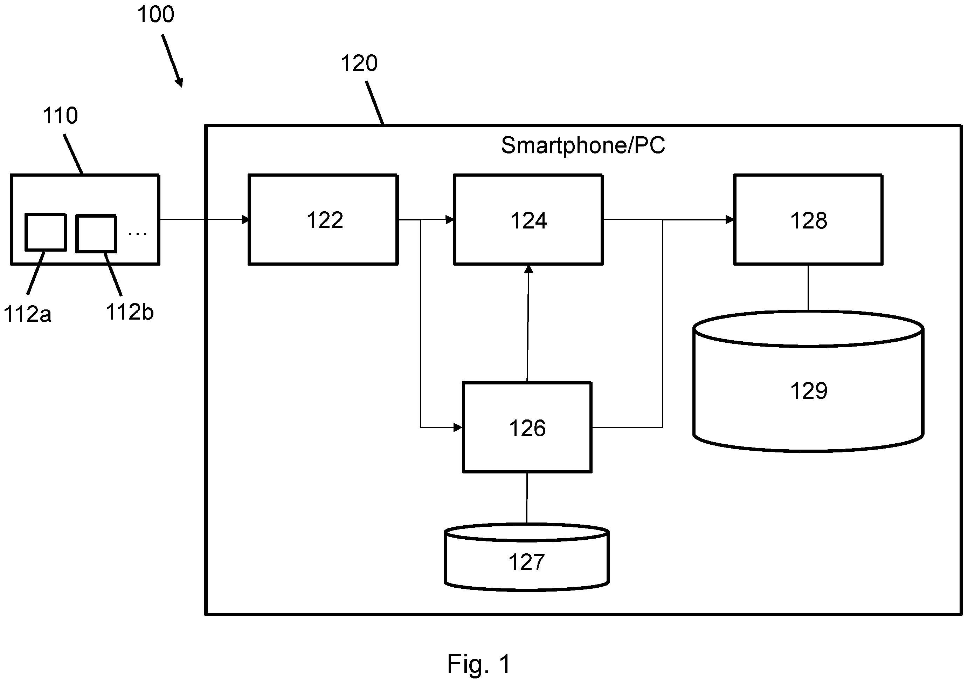

Fig. 1 shows a schematic of a system 100 for performing postural evaluation according to a first example of the present invention. The system 100 comprises a sensor arrangement 110 and a computing device 120 (e.g., a smartphone, personal computer, server, or cloud computing environment) communicatively connected to the sensor arrangement 110.

The sensor arrangement 110 includes sensors 112a 112b which are usable to obtain data about one or more body parts of a user (not shown). More particularly, the sensors 112a 112b are configured to obtain motion and/or positional data about the one or more body parts of the user, which can be used by the computing device 120 to determine both the posture of the user and the task being performed by the user (as described below). The number of sensors is not limited to two however, and any number may be used as indicated by the '...' in Fig. 1.

In the present example, the sensors 112a 112b are Inertial Measurement Unit (IMU) sensors that are attached to different respective body parts of the user, either directly or indirectly through an item of clothing being worn by the user. Movement of said respective body parts of the user is then detected by the attached sensors 112a 112b, and used to determine the motion and/or position of said respective body part. In other examples, the sensors 112a 112b are optical sensors (e.g., cameras) which are configured to point at one or more regions of space in which the user is expected to work. In this way, motion and/or positional data about one or more parts of the user may be collected without requiring the sensors 112a 112b to be attached to the user. Of course the system 100 may include a combination of IMU and optical sensors.

The computing device 120 includes a posture determination unit 122, a posture evaluation unit 124, a task determination unit 126 including an action recognition model 127, and a posture optimizer unit 128 including a posture difference threshold database 129. Each of the units 122 124 126 128 may be a block of software contained in a memory (not shown) of the computing device 120, wherein said blocks of software are executable by a processor (not shown) of the computing device 120. Alternatively, each of the units 122 124 126 128 may represent a subsystem within the computing device 120, where each respective subsystem contains a memory storing the software necessary for the performance of the unit's functionality, and a processor for performing said functionality.

As shown by the arrows in Fig. 1, the posture determination unit 122 is communicatively connected to the sensor arrangement 110 as well as the posture evaluation unit 124 and task determination unit 126. Meanwhile, the posture evaluation unit 124 and task determination unit 126 are each respectively further communicatively connected to the posture optimizer unit 128 and the task determination unit 126 is connected to the posture evaluation unit 124 (although in some examples, as discussed below, this connection can be omitted). The arrows shown in Fig. 1 indicate a general direction of data transmission. I.e., data is transferred from the sensor arrangement 110 to the posture determination unit 122, from the posture determination unit 122 to the posture evaluation unit 124 and task determination unit 126, and from the posture evaluation unit 124 and task determination unit 126 to the posture optimizer unit 128.

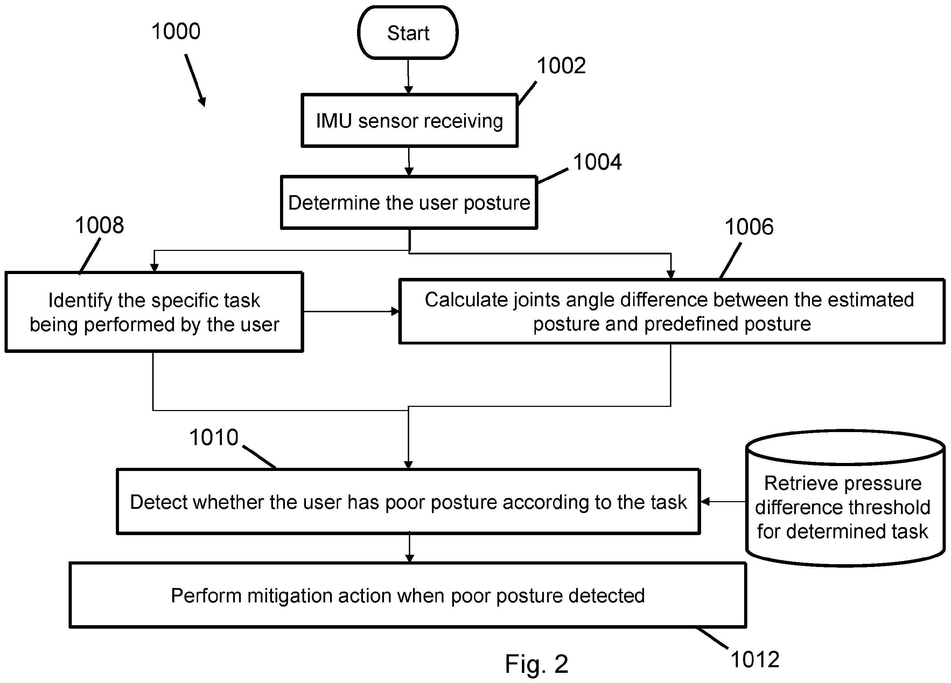

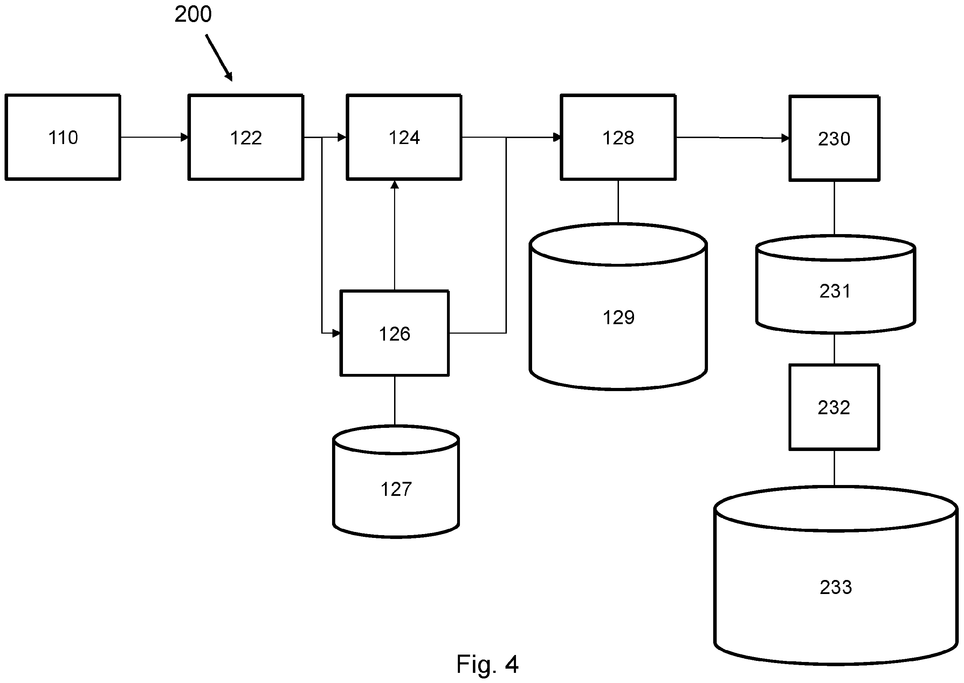

The operation of the system 100 will now be described with reference to Fig. 2, which shows a flowchart 1000 for performing a method of posture evaluation. It will be appreciated that the flowchart 1000 is equally applicable to the systems 200 300 400 shown in Figs. 4-6, which provide specific examples of mitigation actions (see step 1012) that may be performed in response to detecting poor posture as discussed below.

In the first step 1002, data is received from the IMU sensors 112a 112b of the sensor arrangement 110 which are attached to different respective body parts of the user (e.g., the lower arm and upper arm respectively), so that the data relating to the motion and/or positional changes of said body parts can be obtained. The obtaining of motion and/or positional data may continue for a predetermined time, or may continuously until instructed otherwise (e.g., by the user or a component of the system). Alternatively, data may be obtained at discrete intervals of time. The obtained motion and/or positional data is then passed to the posture determination unit 122.

In step 1004, the posture determination unit 122 uses the motion and/or positional data to determine the positions of body parts of the user recorded by the IMU sensors 112a 112b, either in absolute terms or relative to the other body parts of the user that were measured by the IMU sensors 112a 112b. For example, the posture determination unit 122 may determine the angles between said body parts (e.g., the joint angle between the upper arm and the lower arm, which may be referred to as the 'elbow bend'). By taking account of the absolute and/or relative positions of each individual body part recorded by the sensor arrangement 110 in this way, the posture determination unit 122 may determine the posture of the user. Said determined posture is then passed from the posture determination unit 122 to the posture evaluation unit 124 and the task determination unit 126.

After step 1004, step 1006 and step 1008 are depicted as being performed in parallel (and can be performed simultaneously, or in sequence). This is reflective of the posture evaluation unit 124 and task determination unit 126, whose functions respectively correspond to steps 1006 and 1008, being depicted as arranged in parallel in Fig. 1. However, the present disclosure is not limited in this way, and steps 1004 and 1006 may be arranged in any way between steps 1002 and 1008. For example, step 1004 may be performed between step 1002 and step 1006, or step 1006 may be performed between step 1002 and 1004.

In step 1006, the posture evaluation unit 124 is configured to calculate the difference between the posture determined by the posture determination unit 122 in step 1004 and a predetermined template posture, said predetermined template posture being saved in a memory (not shown) forming part of either the posture evaluation unit 124 specifically or the computing device 120 generally. In the present example, this difference is an angular deviation between the calculated joint angles of the determined posture and the predetermined template posture. The determined difference or differences between the two sets of joint angles are then passed to the posture optimizer unit 128.

In some examples of the system 100, there is a single predetermined template posture. In other words, the system 100 assumes that the ideal posture will be the same for all tasks determined by the task determination unit 126 in step 1008 (discussed below). However, in other examples, there may be a plurality of predetermined template postures (e.g. stored in the posture evaluation unit 124), each linked to a different task. In this example, the task determination unit 126 may first determine the task, and then output the determined task to the posture evaluation unit 124 (as indicated by the arrow in Fig. 1 between 126 and 124, and similarly the arrow between steps 1008 and 1006). The posture evaluation unit 124 may then first select a particular predetermined template posture of the plurality of predetermined template postures corresponding to the determined task, before proceeding to determine the difference between the determined posture received from the posture determination unit 122 and the selected predetermined template posture. The selection of the predetermined template posture corresponding to the determined task may include selecting the predetermined template posture of the plurality which most closely matches the determined posture (e.g., has the smallest overall deviation). Alternatively, any number of fuzzy-search type methodologies known *per se* in the art may be utilised to find the predetermined template posture which most closely corresponds to the determined posture. Further alternatively, the system may maintain a database of predetermined template postures with preexisting associations to a given task (and so selection is performed by identifying the predetermined template posture corresponding to the identified task).

In step 1008, the task determination unit 126 is configured to determine the task being performed by the user using a pretrained action recognition model 127. The task may be, for example, an administrative task such as the user working on a document on a computer at a desk, or may be a physical task such as screwing in a screw.

The pretrained action recognition model 127 has been trained using historical motion and/or positional data linked to corresponding tasks. The pretrained action recognition model can comprise a stored database of predetermined tasks with corresponding indicative posture, motion and positional data values or fields (e.g., trained features). As a result, when the action recognition model 127 is then presented with the posture determined by the posture determination unit 122 in step 1004, and/or the motion and positional data captured by the sensor arrangement 110 in step 1002 (said motion and positional data being passed from the posture determination unit 122 to the task determination unit 126 between steps 1002 and 1006), it is able to determine the task that is most closely matched to the presented data, and return this task as the task being performed. Said determined task is then passed from the task determination unit 126 to the posture optimizer unit 128.

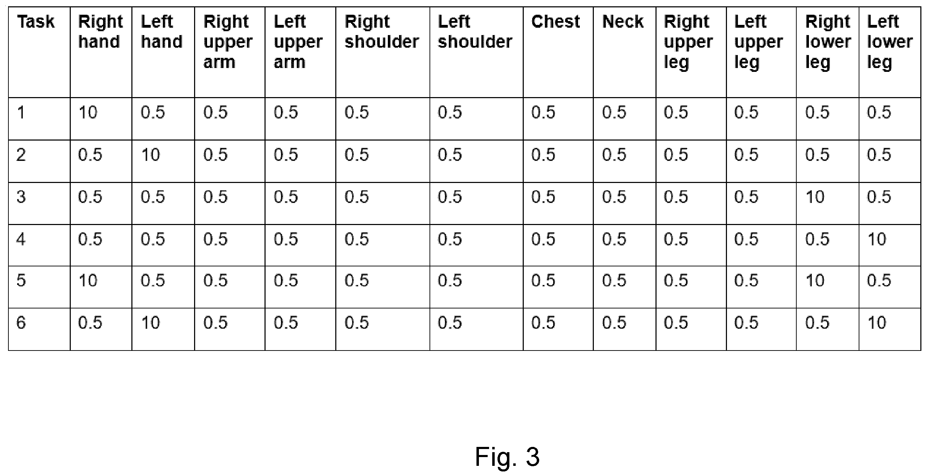

In step 1010, the posture optimizer unit 128 detects whether or not the determined posture of the user is poor using the posture difference threshold database 129. Said posture difference threshold database 129 stores tasks along with a corresponding predetermined posture difference threshold which indicates the level of acceptable divergence in posture from the predetermined template posture for that particular task (although in some examples there may be a single predetermined posture difference threshold which is applied irrespective of the determined task being performed). Examples of predetermined posture difference thresholds for six different tasks are given in Fig. 3, which is a table showing the acceptable level of deviation in absolute or relative position of various body parts in the determined posture compared to the absolute or relative position of the same body parts in the predetermined template posture. The posture optimizer unit 128 scans through the posture difference threshold database 129 for the determined task, and then retrieves the predetermined posture difference threshold matching said task.

The posture optimizer unit 128 then compares the difference/differences measured between the determined posture and the predetermined template posture determined by the posture evaluation unit 124 in step 1004 to the retrieved predetermined posture difference threshold. If the difference measured between the determined posture and the predetermined template posture by the posture evaluation unit 124 in step 1004 is less than the retrieved predetermined posture difference threshold, no further action is taken. However, if the difference measured between the determined posture and the predetermined template posture by the posture evaluation unit 124 in step 1004 is greater than the retrieved predetermined posture difference threshold, step 1012 is performed, in which the posture optimizer unit 128 performs a mitigation action configured to improve the user's posture either directly or indirectly. Examples of mitigation actions that may be performed are described in relation to Figs. 4, 5 and 6 below.

By performing steps 1002-1012, the system 100 is able to take a more holistic view of the user's posture, and provide more accurate and tailored corrective action as a result.

Fig. 4 shows a system 200 for posture evaluation according to a second example of the present invention.

The system 200 is similar to the system 100 of Fig. 1, and like components are given like reference numerals. In particular, as with the system 100, the system 200 includes a sensor arrangement 110 (which may comprise one or more sensors, though these are not shown in Fig. 4), a posture determination unit 122, a posture evaluation unit 124, a task determination unit 126 including a pretrained action recognition model 127, and a posture optimizer unit 128 including a posture difference threshold database 129, all of which perform the same functions as their counterpart components in Fig. 1. In addition, the various units 122 124 126 128 may once again be included on a computing device, as they were in Fig. 1, though this is not shown in Fig. 4. The additional components of the system 200 described below may also be included on said computing device.

In addition to the components of system 100, the system 200 further comprises a chatbot unit 230 including an LLM (large language model) 231, which is configured to generate posture warnings to the user when the system 200 determines that mitigation action is necessary. More specifically, the chatbot unit 230 is communicatively connected to the posture optimizer unit 128. When the posture optimizer unit 128 determines that a difference between a determined posture and a predetermined template posture is greater than the task-specific predetermined posture difference threshold (as described in relation to Figs. 1 and 2), the posture optimizer unit 128 informs the chatbot unit 230 that mitigation action is necessary and passes on the determined difference between the determined posture and the task-specific predetermined posture difference threshold. The chatbot unit 230 is configured to feed this information through the LLM 231, which will generate an appropriate warning message.

The LLM 231 is trained by an LLM training unit 232, which uses historical training data stored on a coaching database 233. The historical training data may include a plurality of stored warning messages, labelled to indicate in what scenarios each stored warning message is appropriate.

The posture warnings may be a text based message. The text based messages may have different tones, depending on the circumstances prompting the posture warning generation. For example, the warning message may be one of the following eight examples:
1. Gentle Reminder: "Let's sit tall! Stretch your spine and roll your shoulders back for your best posture."
2.Encouraging and Positive: "Great posture leads to great feelings! Time to straighten up and shine."
3. Instructional: "Adjust your back: align your ears over your shoulders and relax them down. Feel the difference?"
4. Motivational: "Remember, every time you correct your posture, you're taking a step towards better health. Let's straighten up!"
5. Health Benefit Highlight: "Straightening up helps reduce back pain and boosts energy. Let's get back in alignment."
6. Friendly Nudge: "Oops, looks like we're slouching a bit. Time for a quick stretch and posture reset, don't you think?"
7. Humorous: "Your back called, and it's asking for a little love. How about we straighten up together?"
8. Interactive: "Quick check: Is your back straight? If not, let's fix that together right now."

However, the chatbot unit 230 is not limited to text based messages, and may instead generate haptic or sound posture warnings.

In some examples, the chatbot unit 230 may follow up the posture warning message by providing tailored guidance as to how the user can improve their posture. For example, the chatbot unit 230 may guide the user toward the predetermine template posture by displaying a visual image of the predetermined template posture, and/or provide one or more recommendations as to how the user should alter one or more individual parts of their body.

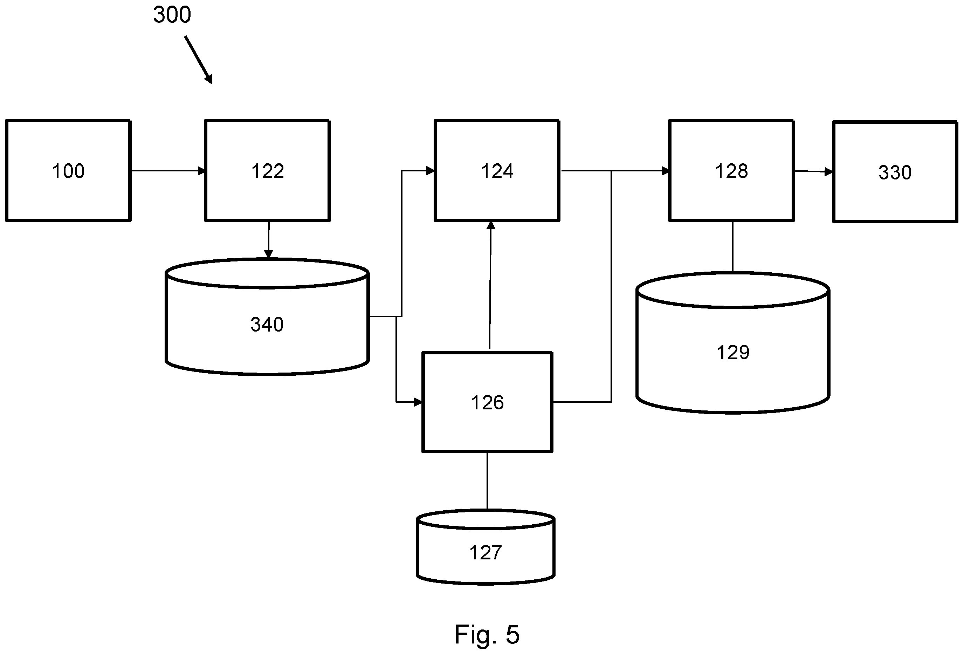

Fig. 5 shows a system 300 for posture evaluation according to a third example of the present invention.

Like the system 200 of Fig. 4, the system 300 is similar to the system of Fig. 1, and like components are given like reference numerals. In particular, as with the system 100, the system 300 includes a sensor arrangement 110 (which may comprise one or more sensors, though these are not shown in Fig. 5), a posture determination unit 122, a posture evaluation unit 124, a task determination unit 126 including a pretrained action recognition model 127, and a posture optimizer unit 128 including a posture difference threshold database 129, all of which perform the same functions as their counterpart components in Fig. 1. In addition, the various units 122 124 126 128 may once again be included on a computing device, as they were in Fig. 1, though this is not shown in Fig. 5. The additional components of the system 300 described below may also be included on said computing device.

However, in place of the chatbot unit 230 of the system 200 of Fig. 4, the system 300 of Fig. 5 instead includes a task reassignment unit 330, which is configured to reassign a series of tasks assigned to the user in order to improve the user's posture when the system 300 determines that mitigation action is necessary.

The task reassignment unit 330 is communicatively connected to the posture optimizer unit 128. When the posture optimizer unit 128 determines that a difference between a determined posture and a predetermined template posture is greater than the task-specific predetermined posture difference threshold (as described in relation to Figs. 1 and 2), the posture optimizer unit 128 informs the task reassignment unit 330 that mitigation action is necessary and passes on the determined difference between the determined posture and the task-specific predetermined posture difference threshold.

The task reassignment unit 330 then retrieves a list of tasks assigned to the user. This list of tasks may have been assigned to the user by the system 300 or an external task assignment unit (not shown), and may include so called 'hard posture tasks' and 'easy posture tasks'. The hard posture tasks being tasks where it is difficult (or more difficult) to maintain a correct posture for extended periods of time, and/or where deviation from a correct posture may quickly cause a large amount of strain on the body. The easy posture tasks may be defined in an opposite way to the hard posture tasks (and so tasks where it is easier to maintain the correct pose than the difficult tasks).

After retrieving the list of tasks, the task reassignment unit 330 determines whether the user's posture can be improved by reassigning the order of the list of tasks. For example, if the user is currently performing a hard posture task, the task reassignment unit 330 may reassign the hard posture task to a later time and reassign an easy posture task to the user to be performed in the present. Alternatively, again if the user is currently performing a hard posture task, and further if the subsequent task or tasks on the list of tasks assigned to the user is/are hard posture tasks, the task reassignment unit 330 may assign one or more easy posture tasks to the user to be performed immediately after the current posture task is concluded and/or between one or more of the upcoming hard posture tasks.

In any of these ways, the user may be provided with effective `breaks' between hard posture tasks, during which they will be able to maintain a posture that is closer to the predetermined template posture more easily. Moreover, the breaks will also give the users the strength necessary to then maintain a good posture when they subsequently return to a hard posture task.

In other examples, the task reassignment unit 330 may reassign tasks from a first list of tasks assigned to a first user to a second list of tasks assigned to a second user when it is determined that mitigation action is necessary for the first user, or vice versa. In this way, the system 300 may ensure both the first user and the second user maintain good posture.

The system 300 further comprises a collected posture database 340, which is communicatively connected between the posture determination unit 122 and the posture evaluation unit 124/task determination unit 126.

The collected posture database 340 is configured to store multiple postures determined by the posture determination unit 122. For example, where the sensor arrangement 110 and posture determination unit 122 are configured to perform their respective functionalities (e.g., obtained motion and/or positional data and determining a posture based on the obtained motion and/or positional data) either continuously or at predetermined intervals of time, a record may be kept of each determined posture. The collected postures in the collected posture database 340, or a subset of the collected postures collected over a predetermined interval of time, may then be used to obtain an average posture of the user, which may be passed through the posture evaluation unit 124, task determination unit 126 and posture optimizer unit 128 to determine whether the user's posture on average is falling outside the task-specific predetermined posture difference threshold.

This is particularly advantageous in the context of the system 300, as the task reassignment type of mitigation action taken in this example may use a more long-term approach to posture improvement that can benefit from taking a more holistic view of the user posture whilst performing any single particular task. In other words, by determining how the user's posture is on average through the performance of a task, it may be more accurately determined whether or not reassignment of the next task or tasks is necessary.

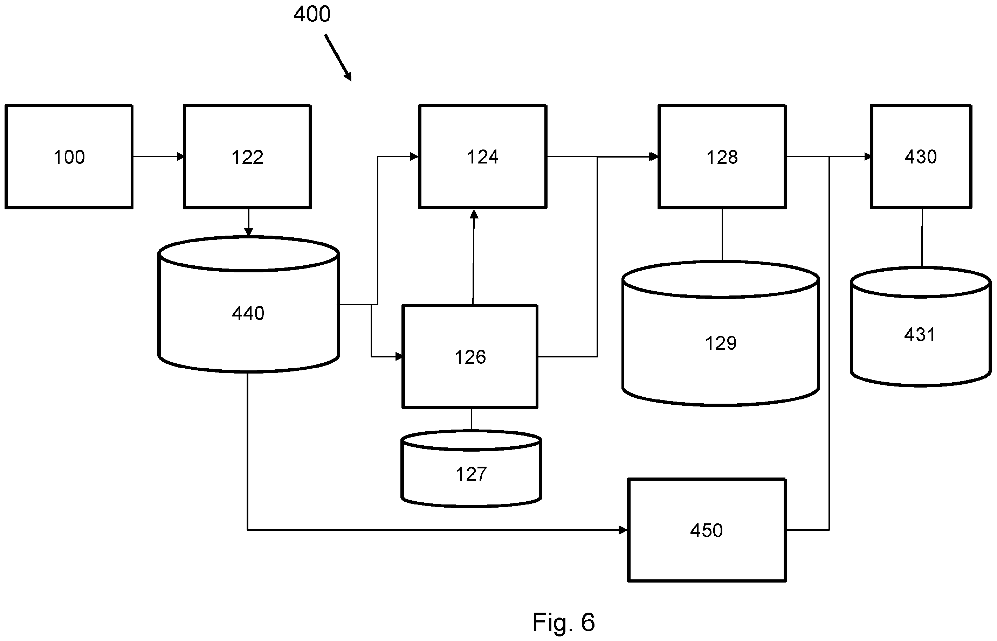

Fig. 6 shows a system 400 according to a fourth example of the present invention.

Like the systems 200 300 of Figs. 4 and 5 respectively, the system 400 is similar to the system of Fig. 1, and like components are given like reference numerals. In particular, as with the system 100, the system 400 includes a sensor arrangement 110 (which may comprise one or more sensors, though these are not shown in Fig. 6), a posture determination unit 122, a posture evaluation unit 124, a task determination unit 126 including a pretrained action recognition model 127, and a posture optimizer unit 128 including a posture difference threshold database 129, all of which perform the same functions as their counterpart components in Fig. 1. In addition, the various units 122 124 126 128 may once again be included on a computing device, as they were in Fig. 1, though this is not shown in Fig. 6. The additional components of the system 400 described below may also be included on said computing device.

However, unlike the system 100 of Fig. 1, the system 400 of Fig. 6 further includes a chatbot unit 430, a collected posture database 440, and a posture improvement feedback unit 450. The chatbot unit 430 is connected to the system 400 in the same way that the chatbot unit 430 is connected to the system 200, and the collected posture database 440 is connected to the system 400 in the same way as the collected posture database 340 of Fig. 5. Additionally, the posture improvement feedback unit 450 is communicatively connected to both the collected posture database 440 and the chatbot unit 430, such that the collected posture database 440 is indirectly connected to the chatbot unit 430 via the posture improvement feedback unit 450.

The chatbot unit 430 is similar to the chatbot unit 230 of Fig. 4, in that it is again configured to provide posture warnings, generated by an LLM 431, to a user when it is determined by the system 400 that mitigation action is necessary. Further, the chatbot unit 430 is again communicatively connected to the posture optimizer unit 128, which informs the chatbot unit 430 when mitigation is necessary and passes on the determined difference between the determined posture and the task-specific predetermined posture difference threshold.

However, in addition to this, the chatbot unit 430 receives further input from the posture improvement feedback unit 450. In particular, the posture improvement feedback unit 450 is configured to compare the latest determined posture (that is, the posture determined by the posture determination unit 122 most recently) to previous determined postures (that is, postures determined by the posture determination unit 122 prior to the most recently determined posture) to determine whether or not the posture is moving closer to the predetermined posture difference threshold. Both the latest determined posture and the previously determined postures being retrievable from the collected posture database 440, which operates in substantially the same way as the collected posture database 340 of Fig. 5 (see above discussion on this). In other words, the posture improvement feedback unit 450 determines whether or not the posture of the user is improving over time.

The posture improvement feedback unit 450 feeds this information into the chatbot unit 430, which may adjust its warning message to the user accordingly. For example, in response to the posture improvement feedback unit 450 determining that the posture is not improving, or has gotten worse, the chatbot unit 430 may issue a more severe posture warning to the user. For example, the chatbot unit 430 may go from a gentle posture warning such as: *"Let's sit tall! Stretch your spine and roll your shoulders back for your best posture"* to an instructional posture warning such as: *"Adjust your back: align your ears over your shoulders and relax them down".* Alternatively, if the posture improvement feedback unit 450 determines that the user posture has improved, the chatbot unit 430 may decrease the severity of its posture warning message, and/or issue a posture warning that is encouraging in tone, instructing the user to keep going.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A system for performing postural evaluation, the system comprising:
a posture determination unit configured to receive motion and/or positional data relating to one or more parts of a user's body to determine a posture of the user;
a task determination unit configured to use the determined posture and/or motion and/or positional data to determine a task being performed by the user;
a posture evaluation unit configured to:
retrieve a predetermined template posture corresponding to the determined task, and
measure a difference between the determined posture of the user and the predetermined template posture; and
a posture optimizer unit configured to perform a mitigation action when the measured difference is greater than a predetermined posture difference threshold.

2. The system of claim 1, wherein the mitigation action includes notifying the user that the measured difference is greater than the predetermined posture difference threshold.

3. The system of claim 2, wherein mitigation action further includes providing guidance to the user as to how to improve their posture.

4. The system of any one of the previous claims, wherein the mitigation action includes reassigning or causing the reassignment of a series of tasks to be performed by the user.

5. The system of any one of the previous claims, wherein the system further comprising a sensor arrangement to obtain the motion and/or positional data.

6. The system of claim 5, wherein the sensor arrangement comprises one or more sensors attached to the user.

7. The system of claim 5 or 6, wherein the sensor arrangement comprises one or more optical sensors.

8. The system of any one of the previous claims, wherein the task determination unit determines the task using a pretrained action recognition model, the pretrained action recognition model having been trained using historical motion and/or positional data linked to corresponding tasks.

9. The system of any previous claim, wherein the posture determination unit and/or the task determination unit are respectively configured to continuously re-determine the posture and task.

10. The system of claim 9, wherein the system is further configured to vary the mitigation action if the measured difference between the determined posture of the user and the predetermined template posture does not decrease over time.

11. The system of any previous claim, wherein the posture determination unit and/or task determination unit are respectively configured to determine the posture and task using data collected over a predetermined interval of time.

12. The system of any previous claim, wherein the predetermined posture difference threshold is one of a plurality of predetermined posture difference thresholds corresponding to different tasks, and the posture optimizer unit is further configured to retrieve the predetermined posture difference threshold corresponding to the determined task.

13. The system of any previous claim, further comprising a chatbot unit configured to provide coaching advice to the user based on the user's motivation.

14. A computer-implemented method for performing postural evaluation, the method comprising the steps of:
receiving motion and/or positional data relating to one or more parts of a user's body from a sensor arrangement;
determining a posture of the user using the motion and/or positional data;
determining a task being performed by the user using the determined posture and/or motion and/or positional data;
retrieving a predetermined template posture corresponding to the determined task;
measuring a difference between the determined posture of the user and the predetermined template posture; and
performing a mitigation action when the measured difference is greater than a predetermined posture difference threshold.

15. A non-transitory computer-readable storage medium containing machine-executable instructions which, when executed on a processor, cause the processor to perform the method of claim 14.

## Amended claims

### Amended claims in accordance with Rule 137(2) EPC.

1. A system for performing postural evaluation, the system comprising:
a posture determination unit configured to receive motion and/or positional data relating to one or more parts of a user's body to determine a posture of the user;
a task determination unit configured to use the determined posture and/or motion and/or positional data to determine a task being performed by the user;
a posture evaluation unit configured to:
retrieve a predetermined template posture corresponding to the determined task, and
measure a difference between the determined posture of the user and the predetermined template posture; and
a posture optimizer unit configured to perform a mitigation action when the measured difference is greater than a predetermined posture difference threshold,
**characterized in that** the task determination unit is configured to return a fallback task as the determined task if the determined posture and/or motion and/or positional data does not substantially match any stored posture and/or motion and/or positional data corresponding to stored tasks,
wherein the fallback task has a fallback predetermined template posture.

2. The system of claim 1, wherein the mitigation action includes notifying the user that the measured difference is greater than the predetermined posture difference threshold.

3. The system of claim 2, wherein mitigation action further includes providing guidance to the user as to how to improve their posture.

4. The system of any one of the previous claims, wherein the mitigation action includes reassigning or causing the reassignment of a series of tasks to be performed by the user.

5. The system of any one of the previous claims, wherein the system further comprising a sensor arrangement to obtain the motion and/or positional data.

6. The system of claim 5, wherein the sensor arrangement comprises one or more sensors attached to the user.

7. The system of claim 5 or 6, wherein the sensor arrangement comprises one or more optical sensors.

8. The system of any one of the previous claims, wherein the task determination unit determines the task using a pretrained action recognition model, the pretrained action recognition model having been trained using historical motion and/or positional data linked to corresponding tasks.

9. The system of any previous claim, wherein the posture determination unit and/or the task determination unit are respectively configured to continuously re-determine the posture and task.

10. The system of claim 9, wherein the system is further configured to vary the mitigation action if the measured difference between the determined posture of the user and the predetermined template posture does not decrease over time.

11. The system of any previous claim, wherein the posture determination unit and/or task determination unit are respectively configured to determine the posture and task using data collected over a predetermined interval of time.

12. The system of any previous claim, wherein the predetermined posture difference threshold is one of a plurality of predetermined posture difference thresholds corresponding to different tasks, and the posture optimizer unit is further configured to retrieve the predetermined posture difference threshold corresponding to the determined task.

13. The system of any previous claim, further comprising a chatbot unit configured to provide coaching advice to the user based on the user's motivation.

14. A computer-implemented method for performing postural evaluation, the method comprising the steps of:
receiving motion and/or positional data relating to one or more parts of a user's body from a sensor arrangement;
determining a posture of the user using the motion and/or positional data;
determining a task being performed by the user using the determined posture and/or motion and/or positional data;
retrieving a predetermined template posture corresponding to the determined task;
measuring a difference between the determined posture of the user and the predetermined template posture; and
performing a mitigation action when the measured difference is greater than a predetermined posture difference threshold,
**characterized in that** the step of determining a task being performed by the user using the determined posture and/or motion and/or positional data comprises a step of:
returning a fallback task as the determined task if the determined posture and/or motion and/or positional data does not substantially match any stored posture and/or motion and/or positional data corresponding to stored tasks,
wherein the fallback task has a fallback predetermined template posture.

15. A non-transitory computer-readable storage medium containing machine-executable instructions which, when executed on a processor, cause the processor to perform the method of claim 14.
